# EUROPEAN PATENT APPLICATION

(11) **EP 1 582 698 A1**
(43) Date of publication of application: **05.10.2005**
(21) Application number: 05251814.9
(22) Date of filing: 23.03.2005
(51) Int. Cl.: F01D 9/04, F01D 5/22, F01D 11/00

(54) **Integral covered nozzle with attached overcover**

(30) Priority: 31.03.2004 US 708909
(71) Applicant: GENERAL ELECTRIC COMPANY, Schenectady, New York 12345 (US)
(72) Inventor: Korzun, Ronald W., New York 12065 (US); Sullivan, Christopher Walter, New York 12020 (US); Bracken, Robert James, New York 12309 (US); Fitts, David Orus, New York 12020 (US)
(74) Representative: Pedder, James Cuthbert

(57) **Abstract**

A method and system of constructing equivalent integral covered blading for a turbine having multiple blades (104) supported by a stator include multiple blade foils; multiple respective cover portions (106) defining a first surface configured to span tips of multiple adjacent blades (104) between tip locations of adjacent blades (104) thereby to form the cover portion (106) portions for adjacent blades (104) and wherein the cover portions (106) associated with each respective adjacent blades (104) include facing sides (118) for adjacent cover portions (106) of adjacent blades (104); and an overcover coupled to a second surface opposite the first surface of the respective cover portions (106), the overcover configured to at least one of stiffen deterministic constraints of the tips and seal against leakage through the facing sides (118) for adjacent cover portions (106).

## Description

The present invention relates to steam turbines and, more particularly, to static carriers, or inner shells containing nozzle blades which direct and accelerate steam for impingement on buckets of a steam turbine.

A steam turbine conventionally employs nozzles formed by a plurality of stationary blades in the steam path which are aerodynamically shaped to receive the steam, smoothly turn it in a desired direction and accelerate it for impingement on turbine buckets. Precision in the steam path is critical to turbine efficiency. The steam must be precisely directed using diaphragm or nozzle blades which are accurately configured and stably supported to avoid power wasting turbulence or off-design flow characteristics.

Some steam turbine design concepts employee diaphragms, which are conventionally formed by inserting opposed ends of blades into cutouts in semi-circular bands known as spacers. The ends of the blades are tack welded to the spacers and outer and inner semi-circular rings are penetration welded to the spacers and to the blades. In order to obtain satisfactory attachment of the blades to the rings, very deep welding is necessary between the rings and spacers so that the weld penetrates far enough in this interface to also contact and attach the blades.

As noted in U.S. Pat. No. 4,288,677, welding defects increase as the weld depth increases. Such welding defects may include, for example, cracks, slag inclusions, lack of complete penetration, etc., which may lead to failure or instability of the nozzle blades. Furthermore, such deep welding tends to distort and to thereby deviate the steam path from the design aerodynamic characteristics and thus produce reduced overall efficiency of the apparatus.

The spacers have typically been relatively thin such as, for example, less than one-quarter inch, since this thickness was the maximum which conventional punching techniques could accommodate. Due to this thinness, the spacers themselves were effective more as positioning agents prior to welding to the inner and outer rings rather than structural members capable of supporting the blades on their own.

A further source of distortion arose due to the fabrication of spacers and blades into semi-circular assemblies. Distortions due to heating and weld shrinkage, material stress relief and tempering tended to distort this subassembly both prior to, and during, mating with the inner and outer semi-circular rings. This distortion could be traced at least in part to the fact that changing stresses in a semi-circular assembly can result in distortion of the semi-circle.

Conventional fabrication techniques have employed a radial mating plane for mating the two halves of the diaphragm. This has customarily required cutting a nozzle blade at each end of each of the semi-circular assemblies due to the overlap of the nozzle blades in the tangential direction. This resulted in relatively flexible blade portions in the steam path which could distort and vibrate to reduce steam flow aerodynamic efficiency.

A second typical steam turbine concept employs individual nozzles inserted into circumferential slots in an inner shell or carrier. This concept can be further divided into 2 different inner diameter tip configurations: 1) peened on covers and 2) integral covered nozzles. Peened on covers group nozzles together by mechanically peening tenons onto the cover. Though mechanically functional, this design provides numerous leakage paths both between the airfoil and cover and between circumferentially adjacent. Alternatively, integral covered nozzles greatly reduces or eliminates these leakage paths. This is very desirable since sealing is a critical part of steam turbine performance.

However, aeromechanic high cycle fatigue (HCF) avoidance and robustness is equally critical for both rotating and non-rotating components in the steampath. Due to assembly and loading retention limitations, integral covered nozzles cannot always guarantee deterministic boundary conditions.

Accordingly, it is desirable to provide a method and product for providing integral cover blades for a turbine which is different to the known integral cover blades and which can provide advantages over other blade cover techniques.

The above discussed and other drawbacks and deficiencies are overcome or alleviated in an exemplary embodiment of the invention by multiple blade foils; multiple respective cover portions defining a first surface configured to span tips of multiple adjacent blades between tip locations of adjacent blades thereby to form the cover portion portions for adjacent blades and wherein the cover portions associated with each respective adjacent blades include facing sides for adjacent cover portions of adjacent blades; and an overcover coupled to a second surface opposite the first surface of the respective cover portions, the overcover configured to at least one of stiffen deterministic constraints of the tips and seal against leakage through the facing sides for adjacent cover portions.

In another embodiment, a method of constructing equivalent integral covered blading for a turbine having multiple blades supported by a stator is disclosed. The method includes attaching multiple blade foils with multiple respective cover portions on a first surface thereof configured to span tips of multiple adjacent blades between tip locations of adjacent blades and wherein the cover portions associated with each respective adjacent blades include facing sides for adjacent cover portions of adjacent blades; and coupling an overcover to a second surface opposite the first surface of the respective cover portions, the overcover configured to at least one of stiffen deterministic constraints of the tips and seal against leakage through the facing sides for adjacent cover portions.

The invention will now be described in greater detail, by way of example, with reference to the drawings, in which:-
Referring now to the drawings wherein like elements are numbered alike in the several Figures:
   FIG. 1 shows an axial cross section of a typical diaphragm construction steam turbine;
   FIG. 2 is a side perspective view of an integral covered nozzle (INC) in accordance with an exemplary embodiment;
   FIG. 3 is a bottom perspective view of three ICN's of FIG. 2 coupled together using an overcover in accordance with an exemplary embodiment;
   FIGS. 4a to 4e illustrate representative interfaces wherein FIG. 4a is a Z-cut shaped interface, FIG. 4b is a rectangular straight cut interface, FIG. 4c is a nested double wing interface, FIG. 4d is a single wing interface, and FIG. 4e is a diagonal interface, wherein the view of all of the blades is from a shaft of the turbine with the bottom of the cover portions being illustrated; and
   FIG. 5 is a top perspective view of the three ICN's joined in FIG. 3 illustrating an interface between corresponding cover portions.

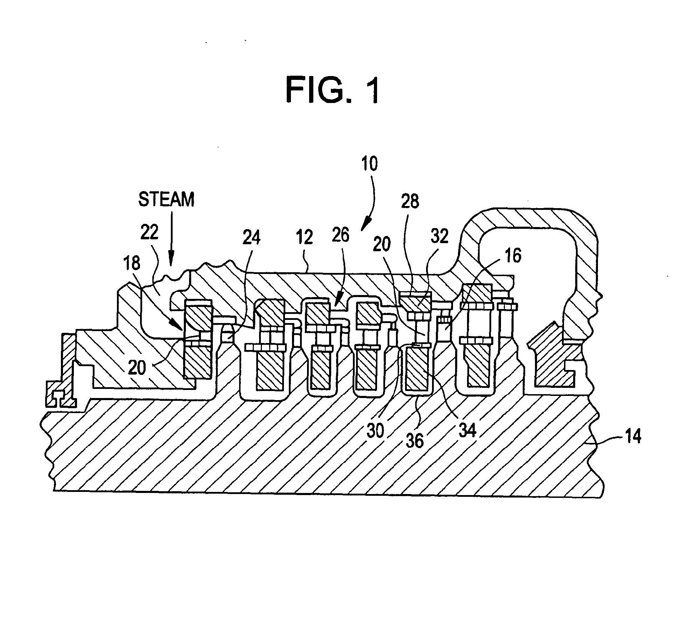

Referring now to FIG. 1, a cross section is shown of an axial flow steam turbine 10 which includes a casing 12 and a rotatable shaft 14. A plurality of turbine buckets 16 are affixed in a conventional manner to shaft 14 for rotation therewith. An inlet diaphragm 18 includes an annular row of blades 20 which accelerate and direct incoming steam from a steam chest 22 onto a first row 24 of turbine buckets.

Additional diaphragms 26 are disposed between pairs of subsequent stages of turbine buckets 16 for redirecting and accelerating the steam emerging from the upstream stage and impinging it at optimum angle and speed on the respective downstream stage.

Each diaphragm 26 includes blades 20 between an outer spacer 28 and an inner spacer 30. An outer ring 32 is affixed to outer spacer 28 and mates in a conventional manner with casing 12. An inner ring 34 is affixed to inner spacer 30 and is suspended spaced from shaft 14. As is conventional, a shaft seal (not shown) may be employed in a seal region 36 at an inner extremity of inner ring 34 to permit shaft 14 to rotate with respect to diaphragm 26 while sealing against axial steam leakage along the rotating shaft.

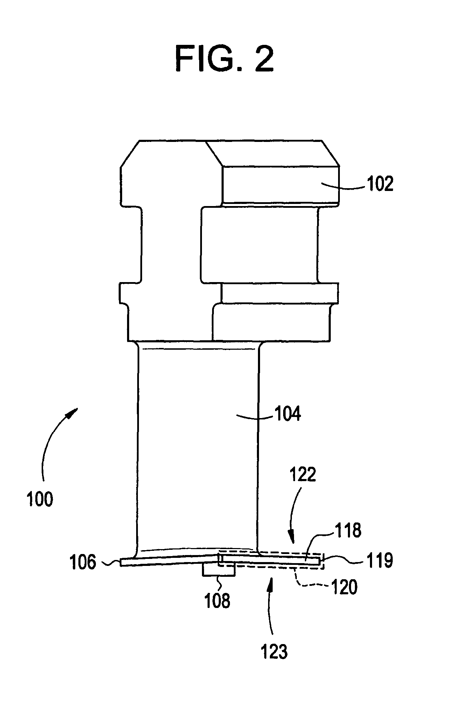

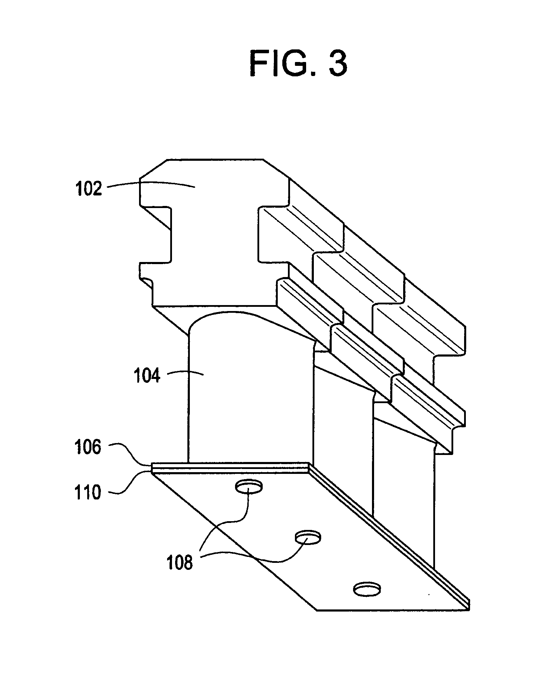

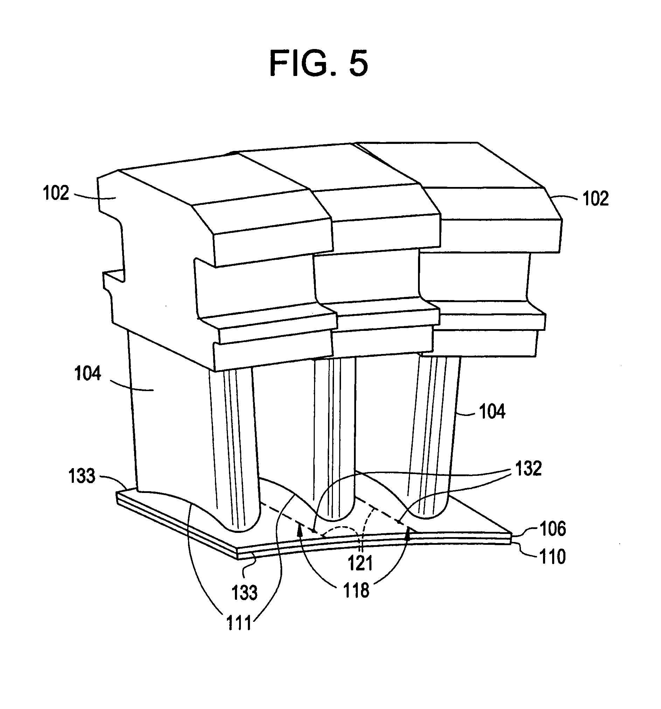

Referring now to FIG. 2, an integral covered nozzle (ICN) 100 is illustrated. Nozzle 100 includes a dovetail segment 102 configured to be operably fixed in a groove in an inner carrier. A foil or nozzle blade 104 is affixed to dovetail segment 102 at one end and is affixed to a cover portion 106 at an opposite end. A tenon 108 extends from cover portion 106 for operable connection with an overcover 110 discussed more fully below with reference to FIGS. 3 and 5. Tenon 108 is preferably riveted to cover portion 106 in an exemplary embodiment, but is not limited thereto.

Referring now to Figure 3, three ICN's 100 are shown coupled together via facing sides 118 and include a overcover or overcover 110 disposed over each of the cover portions 106. It is contemplated that overcover 110 is disposed over multiple ICN's 100 such that multiple means more than one, including two or more ICN'100. Overcover 110 is preferably configured having a thickness less than each respective cover portion 106. Overcover 110 includes apertures positioned therein to align with a tenon 108 extending from each cover portion 106. Each tenon 108 is peened down to retain the overcover 110 which spans across the three nozzles 100 shown. However, any number is contemplated including two nozzles 100 to a number that would complete a ring around shaft 14. Each tenon 108 may be any shape or size and is no longer limited to the shape of the airfoil tip generally shown in FIG. 5 - which typically is restrained to fall within the geometry of the airfoil tip in which it is secured. Other methods of retaining overcover 110 may also be used, such as brazing or welding. If brazing or welding is used for attachment of overcover 110 to cover portions 106, a tenon 108 on cover portions 106 may or may not be used to facilitate this process.

In an exemplary embodiment and referring again to FIG. 2, a material buildup in the form of a weld buildup or, alternatively, braze, plating, spraying or other mechanical means or metallurgical means is applied to each one of the facing sides 118 of the cover portion portions 106. The material buildup 119 is illustrated in FIG. 2 in phantom. Machining is effected to remove excess material 120 from the buildup portion 119 and thereby provide a clean contacting interface 121 between adjacent cover portion portions 106 (See FIG.5). Excess material 120 is on an outer circumferential face portion 122 of the cover portion 106 and also on an inner circumferential face portion 123 of each cover portion 106.

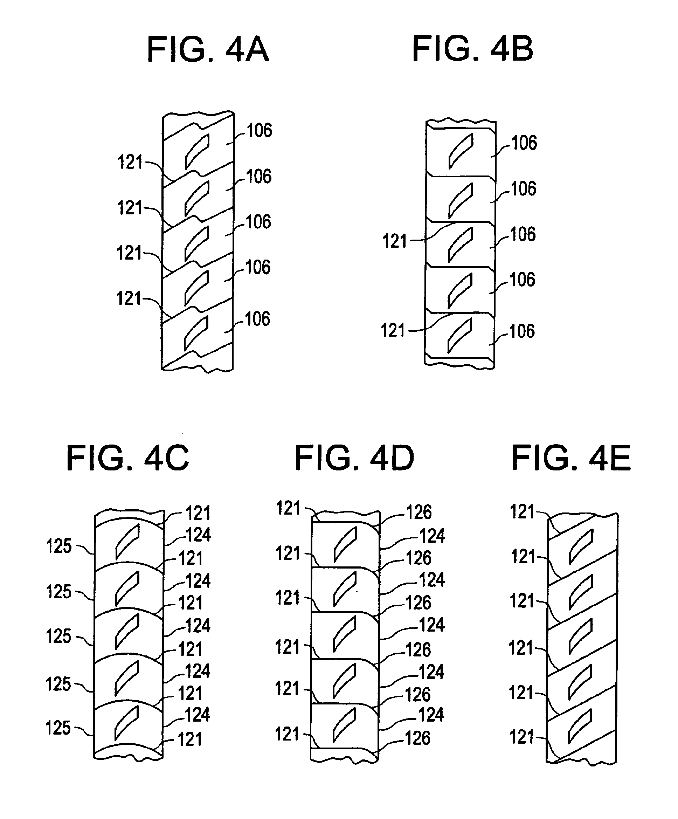

The interface 121 can have different interlocking shapes. In FIG. 4a, interface 121 is illustrated as a Z-cut. In FIG. 4b, the interface 121 is a straight line rectangularly directed relative to the turbine blading and parallel to the rotor's rotational axis. In FIG. 4c, the interface 121 is a double wing or nested construction. This is a construction which has curves directed towards both the trailing edge 124 and leading edge 125 of a turbine blade. In FIG. 4d, a single wing design is illustrated such that the interface 121 has only a single curve 126 in the direction of the trailing edge 124. In FIG. 4e, the interface 121 is a diagonally directed line. The interface is of a nature such that a snug fit can be achieved between adjacent cover portion portions 106. In this manner, a contact at the interface line 121 between neighboring blades or nozzles 100 occurs so that if any blade 104 attempts to vibrate, its motion is dampened by the neighboring blade 104. The example of FIG. 4b is likely the most effective structure since, as the blades try to twist due to a nozzle wake frequency, these rectangular shaped cover portion portions cause an increase in pitch as the frequency increases.

Referring to FIG. 5, individual cover portion portions 106 are assembled on blades 104 and then tack welded at 132 to allow the tenons 108 to be riveted without the cover portion portions 106 moving. After the riveting of tenons 108 to the cover portion portions 106 is effected, excess extension material 133 which runs along the edges of each cover portion 106 is removed. In this manner, the tack weld sections 132 are trimmed off on opposite sides (only one side shown in FIG. 5) leaving integral cover portion portions 106 affixed to each blade 104.

In different embodiments of the invention, integral covered blading can be constructed initially in this manner. Thus, cover portion portions 106 can be affixed to the tips 111 of each blade 104 by suitable welding or brazing. Thereafter, each blade structure constitutes an equivalent integral covered nozzle.

Making use of an overcover on top of an integral covered nozzle (ICN) serves a dual purpose. The overcover not only groups the nozzles to stiffen and maintain deterministic tip constraints, the overcover also seals any leakage that may occur between adjacent integral covered nozzles thorough interface 121.

## Claims

1. A multiple group of blades (20) for an integral covered nozzle (100) of a turbine (10) comprising:
multiple blade foils;
multiple respective cover portions (106) defining a first surface configured to span tips (111) of multiple adjacent blades (104) between tip locations of adjacent blades (104) thereby to form the cover portion (106) portions for adjacent blades (104) and wherein the cover portions (106) associated with each respective adjacent blades (104) include facing sides (118) for adjacent cover portions (106) of adjacent blades (104); and
an overcover (110) coupled to a second surface opposite said first surface of said respective cover portions (106), said overcover (110) configured to at least one of stiffen deterministic constraints of said tips (111) and seal against leakage through said facing sides (118) for adjacent cover portions (106).

2. Blades (104) as claimed in claim 1 wherein each of said multiple respective cover portions (106) include a tenon (108) extending therefrom and through an aperture configured in said overcover (110).

3. Blades (104) as claimed in claim 2 wherein said tenon (108) is one of peened, welded, and brazed with respect to said overcover (110).

4. Blades (104) as claimed in any preceding claim wherein said overcover (110) is configured having a thickness less than each of said multiple respective cover portions (106).

5. Blades (104) as claimed in any preceding claim wherein said overcover (110) is one of welded and brazed to said second surface of said multiple respective cover portions (106).

6. Blades (104) as claimed in claim 5 further comprising a material buildup (119) on at least one facing side of the cover portions (106), the material buildup (119) having been machined to develop an interface (121) between adjacent cover portions (106) of adjacent blades (104).

7. Blades (104) as claimed in claim 6 wherein the material buildup (119) is applied by a selectively mechanical or metallurgical action on both facing sides (118) of the cover portion.

8. Blades (104) as claimed in claim 7 wherein the material buildup (119) is applied between cover portions (106) on all adjacent blades (104) thereby to effect integral covered blading.

9. Blades (104) as claimed in claim 6 including a selectively applied underweld or underbraze between a cover portion (106) and a blade tip thereby to effectively secure the cover portion (106) to the blade (104).
